Europäisches Patentamt

European Patent Office

Office européen des brevets

(19)

(11) Publication number: **0 196 206**
A2

(12) # EUROPEAN PATENT APPLICATION

(21) Application number: **86302137.4**

(22) Date of filing: **24.03.86**

(51) Int. Cl.⁴: **A 61 B 17/58**

(30) Priority: **25.03.85 GB 8507671**

(43) Date of publication of application: **01.10.86**
**Bulletin 86/40**

(84) Designated Contracting States: **AT BE CH DE FR GB IT LI LU NL SE**

(71) Applicant: **Chas F Thackray Limited, P.O. Box HP 171 1 Shire Oak Street, Leeds LS6 2DP West Yorkshire (GB)**

(72) Inventor: **Shearer, John Robertson, Quince Cottage Dean, Bishops Waltham Southampton, SO3 1FY (GB)**

(74) Representative: **Geldard, David Guthrie et al, URQUHART-DYKES AND LORD Tower House Merrion Way, Leeds West Yorkshire LS2 8PA (GB)**

(54) **Fracture fixation assembly.**

(57)  A fracture fixation assembly comprises a fracture plate 10 and location members 22, each of which includes a locating portion 16 and a clamp portion 17. The locating portion is provided with a hole for receiving means, such as a screw 19, for attaching the location device to the bone with the clamping portion of the location member holding the plate against the bone. The fracture plate is devoid of holes and the location members may be such as to allow or prevent relative axial movement between the plate and the location members.

1

# FRACTURE FIXATION ASSEMBLY

This invention relates to a fracture fixation assembly which can be used in the plating method of internal fixation of fractures and in which the "rigid" compression/fixation technique is modified to allow intermittent compression in the longitudinal axis of the bone (dynamic axial compression).

Fracture plates of known kind which are used in this technique at the present time have screw holes drilled in them in order to allow fixation of the plate to the fractured bone. These holes are sometimes offset from the centre which further increases the stress raising action of the hole. In addition, because the fracture is compressed when the plate is applied (compression plating), further compression or intermittent compression of the fracture is only possible due to deformation of the plate.

It is known for plates of this kind to break and this tends to occur if the plate is bent, resulting in fracture of the plate through one of the screw holes adjacent to the fracture. When this happens the continuity of the fixation is completely lost.

As an alternative to a "rigid" internal compression/fixation the use of less rigid, (herein referred to as flexible) plates made, for example, from carbon fibre, has been introduced. Problems do arise when using these plates however. Compared to metals the flexible materials are often weaker and can also break. These flexible plates, like the more rigid plates, are available for use at the moment with holes to allow fixation to the bone by use of screws. Thus these flexible plates have the same weakness as "rigid" drilled compression plates in that the screw holes act as stress-raising points, thus weakening the plates particularly at the screw holes adjacent to the fracture. The rigidity of fixation imparted by flexible plates in all angulatory planes and with regard to torsion is less than with more rigid plates, so that although these plates do allow increased intermittent compression of the fracture, that is, there is less stress protection in the load bearing, there is also less stress protection in angulatory and in torsional movement.

A further disadvantage of a fracture plate having one or more holes therethrough is that the plate may get deformed in the region of the holes either due to the action of the screws bearing against the metal surrounding the holes or as a result of forces applied to the plate either during the surgical procedure or subsequently.

The fracture fixation assembly according to the present invention is intended to overcome some of the disadvantages of "rigid" and flexible plates of the kinds referred to above.

According to the present invention a fracture fixation assembly comprises a fracture plate and two or more location members, each of which has a clamp portion shaped to engage the fracture plate and a location portion provided with means for securing it to the bone with which the assembly is to be used.

The location member can be arranged to act to locate the fracture plate on at least one portion of the fractured bone and allow it to move longitudinally in relation thereto to permit intermittent compression of the fracture, and if desired the plate can move longitudinally in relation to two or more portions of the fractured bone.

Preferably therefore the fracture plate has no holes or openings in it.

Preferably the assembly is provided with location members which allow relative longitudinal movement between the fracture plate and said location members.

Preferably the assembly is also provided with location members which are shaped so as to inhibit relative longitudinal movement between the fracture plate and said location members. Accordingly, a preferred fracture fixation assembly will be provided with at least two types of location members, a first type which allows relative longitudinal movement as above mentioned and a second type which inhibits such movements. Use of the first type permits intermittent compression of the fracture as mentioned above, whereas the second type of location member allows the bone to be maintained in axial compression or distraction across the fracture applied at the time of surgery.

Preferably the location members are such that they may be located in any axial position relative to the plate.

Preferably the location members are such that they may be located at either side of the plate.

Location portions of the location members can be provided with openings to receive retaining screws or pins and these openings can be tapered.

In a preferred form the fracture plate has a generally curved cross-sectional outer surface and the inner surface may be formed with spaced apart sharp edges for engagement with and location on the bone.

With this arrangement the sharp edges are preferably formed on the inner side edges of the plate and the inner surface of the plate can be concave.

Longitudinally spaced apart stops can be provided on the outer surface of the plate and this outer surface may be provided with location means shaped to receive and locate the inner surface of the clamp portion of the location member.

With this arrangement the outer surface of the plate can be provided with at least one longitudinally projecting rib which can be located in a cooperating groove or grooves on the inner surface of the clip portion of the location member. Alternatively, the outer surface of the plate may be provided with at least one longitudinally projecting groove, into which a cooperating rib or ribs on the inner surface of the clip portion of the location member can locate.

The plate may also be provided with numerous serrations along its length which will locate with a modified locating member. In this way it is possible to inhibit axial compression during weight bearing which may be necessary when treating comminuted fractures.

This feature may also be used to maintain axial compression across the fracture which would otherwise be lost due to the dynamic loading mechanism.

An alternative form of fixation can be provided at one end of the fracture plate by a transversely extending extension or extensions.

Thus, the extension or extensions can be provided with location openings and each of these may be provided with an insert having a tapered hole therein to receive a screw or pin.

The invention can be performed in many ways and some embodiments will now be described by way of example and with reference to the accompanying drawings in which:-

Figure 1 is a diagrammatic cross-sectional view showing a compression plate in position on a bone;

Figure 2 is a front elevation of a pinned compression plate in position on a bone, showing the effect of angulatory forces;

Figure 3 is a diagrammatic side elevation of a pinned compression plate showing the effect of angulation of the bone in the plate of the thickness of bone plate showing the plate acting as a hinge on the convex side of the bone;

Figure 4 shows a similar construction to Figure 3 with the plate on the concave side of the bone showing a similar phenomenon;

Figure 5 shows a side view of a plate according to the present invention;

Figure 6 is a plan view of the plate shown in Figure 5;

Figure 7 is a cross-sectional end elevation on the line VII-VII of Figure 6;

Figure 8 is a side-elevation of a location member for use with the present invention;

Figure 9 is a plan view of the member shown in Figure 8;

Figure 10 is a diagrammatic cross-section of a fracture fixation assembly in use;

Figure 11 is a diagrammatic view of the assembly in use employing two or more location members;

Figure 12 is a plan view of an alternative plate construction;

Figure 13 is an end elevation of the plate construction shown in Figure 12; and,

Figure 14 is a perspective view of an alternative plate type and an alternative type of clip.

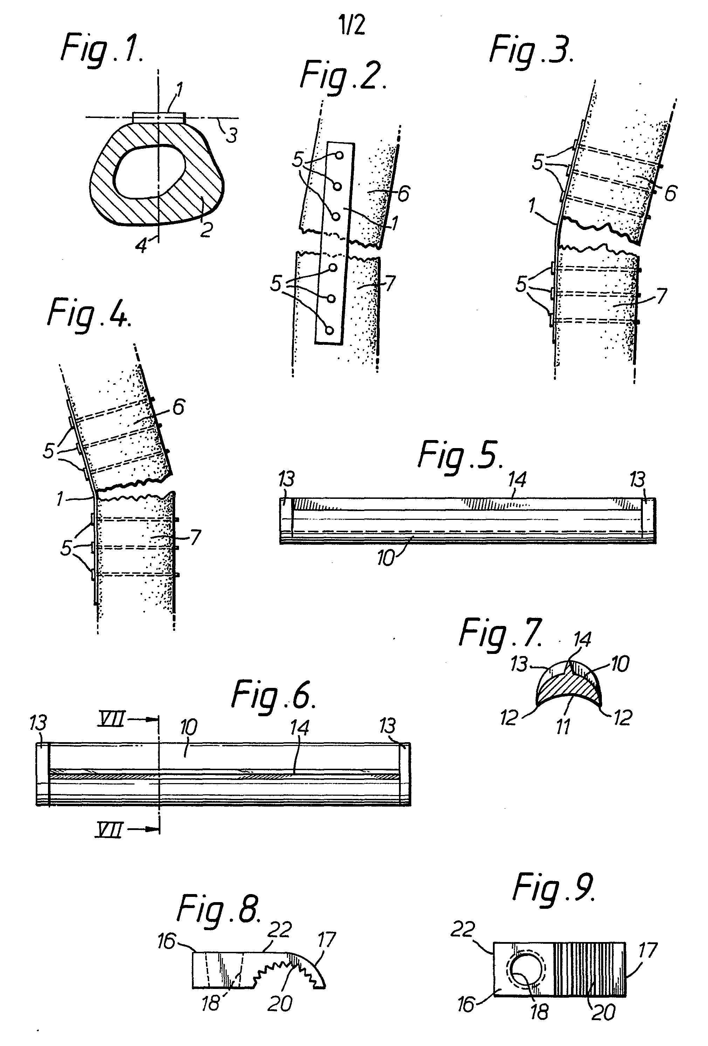

Figure 1 shows a compression plate 1 in position on a bone 2. The plane of the width of the plate is indicated by broken line 3 and the plane of the thickness of the plate is indicated by broken line 4.

Figures 2, 3 and 4 show a plate 1 of known type held by pins or screws 5 in position on a fractured bone 2, the upper part of the fractured bone being indicated by reference numeral 6 and the lower part by reference numeral 7.

When a bone is plated in this manner the bone takes some of the load when angulatory forces are applied to it in the plane of the width of the plate. Thus, the plate tends to bend and act as a hinge and the fractured edges on the concave side of the connection come into contact as shown in Figure 2 thus tending to relieve the plate.

When the bone is angulated in the plane containing the thickness of the plate then, if the plate is on the convex side of the bone a similar phenomenon occurs, that is, the fractured edges are loaded and relieve the stress applied to the plate as shown diagrammatically in Figure 3. If the plate is angulated in the opposite direction however, as shown in Figure 4, the only means of reducing the angulatory load on the plate would be the pressure of callus forming on the bone. In the earlier stages of fracture repair this callus is non-existant so that all the angulatory load is taken by the plate. This is the plane in which known types of plate tend to break.

A plate for use in a fracture fixation assembly according to the present invention is shown in Figures 5, 6 and 7. This plate is a straight metal plate which can be made in stainless steel but can be made in any other material with mechanical properties suitable for fracture fixation and biological properties which make it suitable for insertion into human tissue. The plate is available in various lengths widths and heights as required. The shape of the plate is however similar in all its various sizes and is substantially semi-circular on its convex outer surface 10, as is most clearly shown in Figure 7. The inner surface 11 is an arc of a circle and where the two surfaces meet, that is the inner side edges of the plate, they form sharp edges 12 which are spaced apart and are for engagement and location on the bone.

Longitudinally spaced apart stops 13 are provided on the outer surface by semi-circular abutments.

The sharp edges 12 will bite into the bone and help to reduce torsional movement.

The outer surface 10 of the plate is provided with location means to receive and locate the inner surface of the clamp portion of the location members which are described hereunder and this location means is in the form of at least one longitudinally projecting rib 14 on the outer surface 10.

A number of location members are provided to engage the fracture plate and locate it on the bone and a suitable member in the form of a clamping clip 22 is shown in Figures 8 and 9. This clip comprises a location portion 16 and a clamp portion 17.

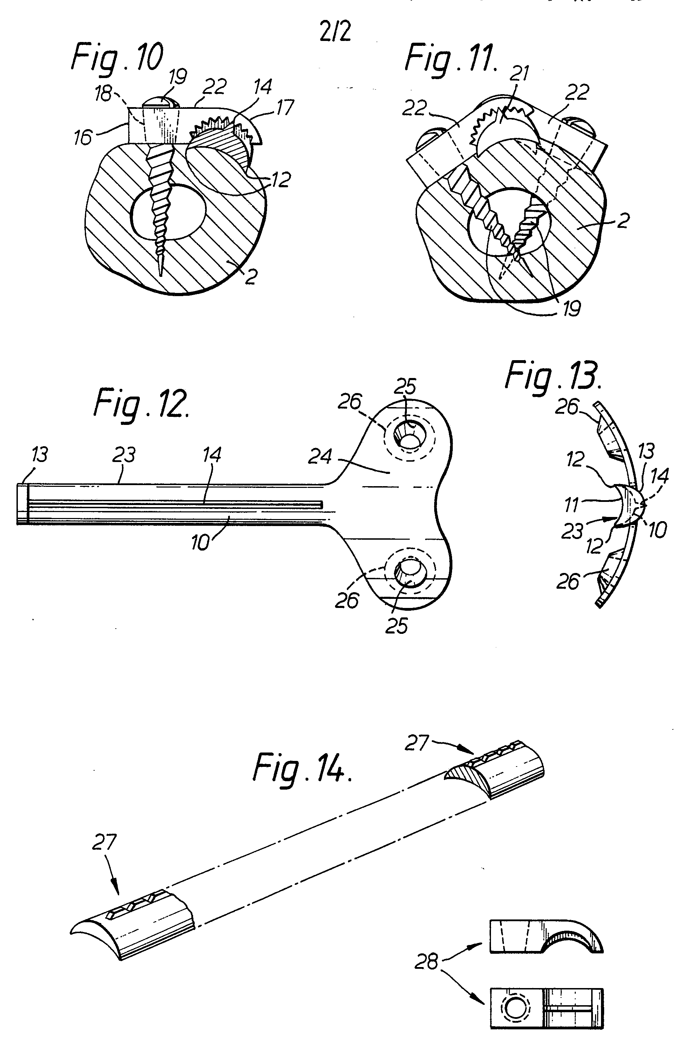

The location portion 16 may be provided with a tapered opening 18 to receive a tapered screw 19, as shown in Figure 10.

The location portion 17 of the clip 22 is in the form of a semi-circular hook, the inner surface of which is provided with a series of ridges 20 shaped to fit over the rib 14 on the plate.

The size of the clips may vary depending on the size of the plate.

The parts are assembled in the manner shown in Figure 10, that is the clamping clips are used to clamp the plate in position, the appropriate ridges 20 engaging the rib 14 to locate the plate against sideways movement and the spaced apart sharp edges 12 of the plate biting into the bone 2.

The plate will be clipped on both sides of the fracture and a variable number of clips can be used in assocation with each plate. In Figure 11 the plate 21 is shown clamped in position by two or more clamping clips 22.

The size of the screws 19, both in length and diameter, will vary according to the site of insertion and the clips may also vary in size depending on the site of the insertion.

These clips 22 may be made of stainless steel but other biologically mechanically suitable materals may be used and this is also the case with regard to the screws.

Just as the plates and clips will vary in size depending on the size of the bone for which they are intended to internally fix, so the bone screws will vary in size in order to fit the clips, the screws may also vary in length within each size. The general configuration of the screws will be constant but there are several exceptions to this. Screws can also be provided with a lag thread to allow better fixation in the cancellous bone, that is to say they may be provided with no thread adjacent to the head of the screw in order to allow compression of some bone fragments. Moreover, some screws may be provided with a washer in situ around the neck of the screw. The clips 22 may be placed on either side of the plate, as shown in Figure 11, although in some situations all the screws in one or both fragments may be placed on the same side of the plate. This however is a matter for a surgical judgement at the time of insertion.

When the clips are placed in position and the screw is driven home it comes under tension and because of the conical shape of the head of the screw and the screw hole in the clip, some inherent stability is achieved between the screw and the clip. This further improves torsional stability of the assembly as will the fact that the screws can be offset to each other which in itself imparts considerable torsional stability to the fracture, as shown in Figure 11.

Because there are no drill holes supplied in the plate optimal siting of the screws can be achieved by the surgeon.

It will be appreciated that provided the clips 22 are spaced away from the stops 13 on the plate and the appropriate type of clip is used, some longitudinal movement of the plate is possible although there is no sideways or torsional movement and thus intermittent compression of the fracture by allowing the patient to weight bear is achieved with ease. This means that as soon as the patient is allowed to weight bear intermittent compression of the fracture, (which is a recognised potent stimulus to fracture union) is achieved. Whilst intermittent compression is happening the plate prevents angulation and torsional movement thereby protecting the fracture from angulatory and torsional stress.

Where there are fractures near the end of a long bone a modified form of plate can be used as shown in Figure 12. One end of this plate 23 is provided with a stop 13 and a rib 14 is also formed on it. The other end of the plate however is provided with a portion 24 which may be "butterfly" shaped. This butterfly shaped portion which acts as a transversely extending extension or extensions is provided with two screw holes 25 similar in shape and construction to those available on the clips 22. As a result when screws are applied to the end of the said long bone some inherent angulatory stability is achieved because of the stabilizing effect of the conical fit of the screws in the holes. The remainder of the plate on the other side of the fracture can however still move because it will be clamped in the manner described above and thus intermittent compression of the fracture in the longitudinal axis of the bone is still achieved without angulation and with minimal torsional movement. In order to provide the tapered shape to the holes 25 each hole may be provided with an insert 26 having the tapered hole therein.

The most likely point of breakage of the assembly, if breakage were to occur, would be one or other of the two clips 22 adjacent to the fracture. Even if such a clip did break or a screw pull out, total loss of control of fixation of the fracture would not occur because of the presence of the remaining clips in that bone fragment.

Figure 14 shows an alternative plate type which has serrations 27 along its curved surface which locate with an alternative type of clip 28 to prevent axial sliding of the plate with respect to the clip or clips.

The shape of the serrations will preferably be as shown with a largely triangular form, although any form including square or sawtooth forms is possible.

CLAIMS

1.   A fracture fixation assembly characterised in that it comprises a fracture plate (10) and two or more location members (22), each of which has a clamp portion (17) shaped to engage the fracture plate and a location portion (16) provided with means for securing it to the bone with which the assembly is to be used.

2.   A fracture fixation assembly according to claim 1 characterised in that the fracture (10) plate is substantially devoid of holes therethrough or openings thereinto.

3.   A fracture fixation assembly according to claim 1 or claim 2 characterised in that the assembly is provided with location members (22) which are shaped so as to allow relative longitudinal movement between the fracture plate and said location members.

4.   A fracture fixation assembly according to any of the preceding claims characterised in that there is included location members (28) which are shaped so as to inhibit relative longitudinal movement between the fracture plate and said location members.

5.   A fracture fixation assembly according to any of the preceding claims characterised in that the location member (22, 28) is such that it can be located in any axial position relative to the plate.

6. A fracture fixation assembly according to any of the preceding claims characterised in that the location members (22, 28) are such that they may be located at either side of the plate.

7.   A fracture fixation assembly according to any of the preceding claims characterised in that the fracture plate (10) has a generally curved cross-sectional outer surface and the inner surface (11) may be formed with spaced apart edges for engagement with and location on the bone.

8. A fracture fixation assembly according to any of the preceding claims characterised in that the means (19) for securing the location member to the bone is a screw.

9. A fracture fixation assembly according to any of the preceding claims characterised in that the plate is provided with one or more continuous or intermittent longitudinal ridges (14, 27) extending along its outer surface.

10. A fracture fixation assembly according to any of claims 1 to 8 characterised in that the fracture plate is provided with one or more continuous or intermittent longitudinal grooves extending along its outer surface.

1/2

**Fig.1.**

**Fig.2.**

**Fig.3.**

**Fig.4.**

**Fig.5.**

**Fig.6.**

**Fig.7.**

**Fig.8.**

**Fig.9.**

0196206

2/2

**Fig. 10**

**Fig. 11.**

**Fig. 12.**

**Fig. 13.**

**Fig. 14.**